# DEMANDE DE BREVET EUROPEEN

(11) **EP 1 174 091 A2**
(43) Date de publication de la demande: **23.01.2002**
(21) Numéro de dépôt: 01810690.6
(22) Date de dépôt: 11.07.2001
(51) Int. Cl.: A61B 17/16

(54) **Fraise chirurgicale**

(30) Priorité: 20.07.2000 CH 143500
(71) Demandeur: PRECIMED S.A., 2534 Orvin (CH)
(72) Inventeur: Lechot, André, 2534 Orvin (CH)
(74) Mandataire: Meylan, Robert Maurice

(57) **Abrégé**

Fraise destinée à la chirurgie, constituée d'un corps creux de révolution muni de quatre bras radiaux perpendiculaires entre eux de manière à former une croix pour la fixation de la fraise sur un porte-fraise. La croix formée par les bras radiaux est constituée d'une première barrette diamétrale (1), d'une goupille (2) traversant la première barrette en son milieu et faisant saillie de chaque côté de cette barrette, et de deux barrettes radiales (3, 4) présentant un trou axial par lequel chacune de ces barrettes radiales est chassée sur la goupille.

Cette construction, simple à réaliser, permet de supprimer des soudures et les problèmes de nettoyage inhérents à ces soudures.

## Description

L'invention a pour objet une fraise destinée à la chirurgie, constituée d'un corps creux de révolution muni de quatre bras radiaux perpendiculaires entre eux de manière à former une croix pour la fixation de la fraise sur un porte-fraise.

Une telle fraise est décrite et représentée dans les brevets EP 0 704 191 et US 5,658,290. La présence de quatre bras permet une fixation centrée et stable de fraises de différents diamètres sur le porte-fraise décrit dans ces mêmes brevets. Jusqu'ici, la fixation des bras entre eux s'est faite par soudage conventionnel, c'est-à-dire avec apport de matière. Or, on a constaté un noircissement de la soudure lors de la stérilisation de la fraise. Un tel noircissement est inacceptable pour un outil chirurgical. En outre, la soudure présente une certaine porosité qui pose un problème de nettoyage de l'instrument.

Ces inconvénients pourraient être éliminés par une soudure au laser, c'est-à-dire sans apport de matière. Une telle soudure nécessite toutefois un usinage préalable précis et coûteux des parties à assembler. Une telle solution doit donc être écartée pour des raisons économiques.

La présente invention a pour but de réaliser un assemblage sans soudure de la croix par des moyens économiques et faciles à mettre en oeuvre.

La fraise selon l'invention est caractérisée en ce que la croix formée par les bras radiaux est constituée d'une première barrette diamétrale, d'une goupille traversant la première barrette en son milieu et faisant saillie de chaque côté de cette première barrette et de deux barrettes radiales présentant un trou axial par lequel chacune de ces barrettes est chassée sur la goupille.

La goupille est un élément bon marché. Les trou axiaux sont faciles à réaliser et le chassage est une opération simple, peu onéreuse. La fixation obtenue est très solide.

Le dessin annexé représente, à titre d'exemple, un mode d'exécution de l'invention.

La figure 1 est une vue en plan de la croix de fixation de la fraise.

La figure 2 est une vue de cette croix de fixation selon l'axe de la barrette diamétrale.

La figure 3 est une vue en coupe de la fraise selon un plan de symétrie perpendiculaire à la barrette diamétrale de la croix.

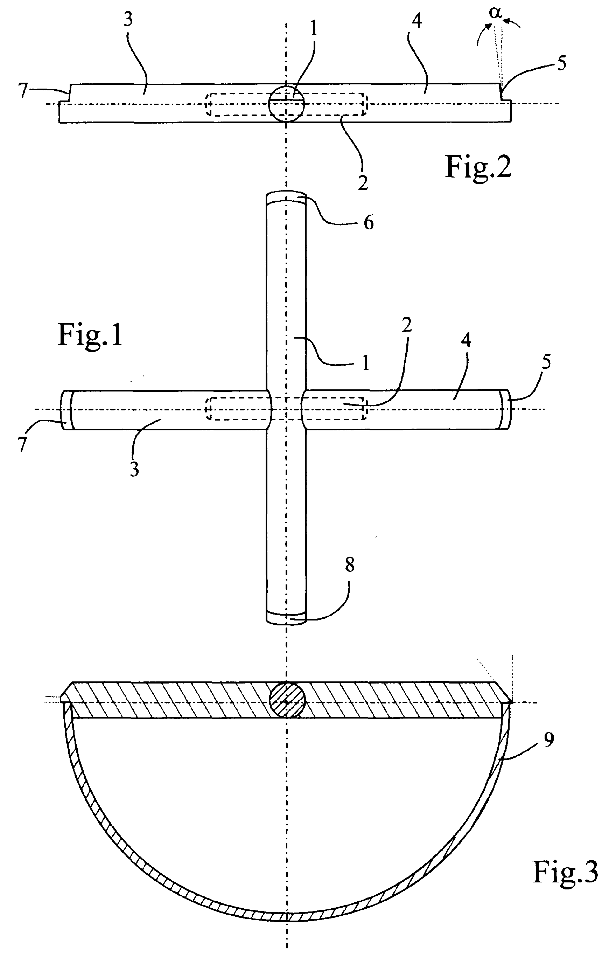

La croix de fixation représentée aux figures 1 et 2 est constituée d'une barrette diamétrale 1 percée d'un trou perpendiculaire à son axe, trou dans lequel est enfilée une goupille cylindrique 2 de longueur telle qu'elle fait largement saillie de chaque côté de la barrette 1. Sur cette goupille, de chaque côté de la barrette 1, sont chassés deux bras radiaux 3 et 4 perpendiculaires à la barrette 1. A cet effet, les bras 3 et 4 sont percés d'un trou axial borgne de diamètre légèrement inférieur au diamètre de la goupille 2.

Aux extrémités des bras de la croix ainsi formée sont usinées, sur une partie du diamètre de ces bras, des facettes tronconiques 5, 6, 7, 8 situées sur une même surface conique dont l'axe passe par le centre de la croix de fixation. Le diamètre de cette surface conique au niveau des facettes 5 à 8 correspond au diamètre intérieur de la fraise 9 représentée à la figure 3 mesuré dans son plan équatorial de telle manière que la croix de fixation peut être ajustée dans la calotte hémisphérique de la fraise 9 comme représentée à la figure 3. La croix est fixée à la fraise 9 par soudage au laser. Les extrémités de la croix de fixation sont en outre chanfreinées comme ceci est également visible à la figure 3.

A la figure 3, la fraise a été représentée schématiquement par une simple calotte lisse, mais en réalité cette calotte est bien entendu munie de dents comme représenté par exemple dans le brevet US 4,811,632.

La fraise peut, bien entendu, présenter une forme autre qu'hémisphérique, par exemple une forme conique.

## Revendications

1. Fraise destinée à la chirurgie constituée d'un corps creux de révolution muni de quatre bras radiaux perpendiculaires entre eux de manière à former une croix pour la fixation de la fraise sur un porte-fraise, **caractérisée en ce que** la croix formée par les bras radiaux est constituée d'une première barrette diamétrale (1), d'une goupille (2) traversant la première barrette en son milieu et faisant saillie de chaque côté de cette barrette, et de deux barrettes radiales (3, 4) présentant un trou axial par lequel chacune de ces barrettes radiales est chassée sur la goupille.
